# FASCICULE DE BREVET EUROPEEN

(11) **EP 1 093 823 B1**
(45) Date de publication et mention de la délivrance du brevet: **18.07.2007**
(21) Numéro de dépôt: 00402885.8
(22) Date de dépôt: 18.10.2000
(51) Int. Cl.: A61L 2/00, B01D 39/16

(54) **Unité de filtration d'une substance virucide**
Filtrationseinheit für eine viruzide Substanz
Filtration unit for a virucidal substance

(30) Priorité: 20.10.1999 FR 9913089
(43) Date de publication de la demande: 25.04.2001
(73) Titulaire: MACO PHARMA, F-59200 Tourcoing (Nord) (FR)
(72) Inventeur: Goudaliez, Francis, 59155 Faches Thumesnil (FR); Verpoort, Thierry, 59420 Mouvaux (FR)
(74) Mandataire: Breese Derambure Majerowicz

(56) Documents cités:
- WO-A-95/16348
- WO-A-98/22151
- US-A- 5 858 641

## Description

L'invention est relative à une unité de filtration destinée à éliminer une substance virucide présente dans un fluide biologique.

Elle s'applique typiquement au cas où la substance virucide a été préalablement additionnée à un fluide biologique, notamment du plasma sanguin, destiné à être transfusé à un patient. Cette addition a pour but de faire subir au fluide biologique un traitement d'inactivation virale préalablement à sa transfusion au patient, de sorte à inactiver d'éventuels virus qui contaminent le fluide biologique.

Une technique classique d'inactivation virale du plasma utilise comme substance virucide une substance colorante, par exemple le bleu de méthylène ou l'un de ses dérivés.

Le principe de cette technique repose sur des réactions photochimiques entre la substance colorante et l'ADN ou l'ARN viral éventuellement présent dans le fluide biologique. L'exposition à la lumière de la substance colorante entraîne une réaction photochimique qui transmet de l'énergie aux molécules d'ADN et d'ARN de sorte que le virus est inactivé.

Lors de ces réactions photochimiques, la substance colorante n'est pas éliminée de sorte qu'elle reste dans le fluide biologique après l'exposition à la lumière.

Après la mise en oeuvre de cette technique d'inactivation virale, la substance colorante, en quantité très faible, peut être laissée dans le fluide biologique et ainsi être transfusé au patient en même temps que celui-ci.

Cependant, des études récentes semblent montrer la possible toxicité de certaines substances colorantes utilisées, et notamment du bleu de méthylène, lorsqu'elles sont injectées au patient.

De sorte que de nombreux pays demandent l'élimination systématique des substances colorantes préalablement à l'injection du fluide biologique au patient.

Le document US5858641 décrit un procédé pour éliminer le bleu de méthylène du sang ou d'une fraction de sang par passage au travers d'un filtre comprenant une matrice copolymère de poly(alcool vinylique-acétal). La matrice est sous forme de tampons éponges ou de granules.

Dans le document W09822515, une éponge poreuse d'acétate de polyvinyle alcool acétal est utilisée pour éliminer un composé iodé du sang.

L'invention vise donc à proposer une unité de filtration qui permet d'éliminer sensiblement toute la substance virucide présente dans le fluide biologique en laissant sensiblement inchangée la composition du fluide biologique lors de la filtration.

A cet effet, l'invention a pour objet une unité de filtration destinée à éliminer une substance virucide présente dans un fluide biologique, comprenant une enveloppe extérieure munie d'au moins un orifice d'entrée et d'au moins un orifice de sortie, l'enveloppe renfermant un milieu filtrant qui délimite deux compartiments respectivement d'entrée et de sortie de l'unité de filtration, dans laquelle le milieu filtrant est réalisé à partir d'au moins un matériau hydrophile sous la forme d'un non tissé poreux apte à absorber et/ou adsorber la substance virucide.

Selon une réalisation, la porosité moyenne du milieu filtrant est définie de sorte que la surface de contact entre le fluide biologique et le milieu filtrant soit suffisante pour éliminer sensiblement toute la substance virucide en laissant sensiblement inchangée la composition du fluide biologique lors de son passage à travers le milieu filtrant, à savoir étant comprise entre 1 µm et 15 µm.

En variante, le diamètre moyen des fibres du non tissé poreux est compris entre 0,5 µm et 5 µm.

Le compartiment d'entrée et/ou le compartiment de sortie communique avec l'extérieur de l'unité de filtration par l'intermédiaire d'une tubulure d'entrée respectivement de sortie.

Le matériau hydrophile du milieu filtrant est choisi notamment parmi les matériaux hydrophiles naturellement ou les matériaux, notamment à base de matière plastique, rendu hydrophiles, par exemple parmi les polymères et/ou les copolymères à base de polyester, d'acrylonitrile, de fluorure de polyvinylidène.

Selon une réalisation, le milieu filtrant comprend plusieurs couches de matériau hydrophile, de nature identique ou différente entre eux, de surface de contact identique ou différente entre eux.

Le milieu filtrant possède par exemple une épaisseur comprise entre 1 et 10 millimètres.

Selon une réalisation, l'enveloppe extérieure de l'unité de filtration est rigide.

Selon une autre réalisation, l'enveloppe extérieure de l'unité de filtration est souple.

En variante, l'enveloppe souple est formée de deux feuilles de matière plastique souple assemblée sur leur périphérie, le milieu filtrant étant maintenu dans un cadre souple et étanche délimitant avec le milieu filtrant les compartiments d'entrée et de sortie de l'unité de filtration.

Le cadre souple est, par exemple, formé de deux feuilles souples ajourées entre elles entre lesquelles le milieu filtrant est placé, les feuilles souples étant fixées entre elles au niveau de la périphérie du milieu filtrant et également avec les feuilles formant l'enveloppe extérieure, au niveau de la périphérie de l'enveloppe extérieure de l'unité de filtration

La fixation des feuilles formant le cadre souple est alors un cordon de soudure réalisé à travers le milieu filtrant.

Selon une réalisation, le compartiment de sortie est dégagé du milieu filtrant par la présence d'un ou plusieurs joncs d'écartement disposés entre le milieu filtrant et l'enveloppe extérieure souple, à l'intérieur du compartiment de sortie.

Le ou les joncs d'écartement sont réalisés à partir de tubulures souples soudées par exemple au niveau de la paroi interne de la feuille de l'enveloppe extérieure.

D'autres objets et avantages de l'invention apparaîtront au cours de la description qui suit en référence aux dessins annexés.

La figure 1 représente, en vue de coté et en coupe longitudinale, un mode de réalisation de l'unité de filtration comprenant une enveloppe extérieure souple.

La figure 2 représente, en vue de face et en coupe longitudinale partielle, le mode de réalisation de la figure 1, montrant en particulier l'assemblage du milieu filtrant dans un cadre souple.

La figure 3 représente, en vue de dessus et en coupe transversale, le mode de réalisation de la figure 1, montrant en particulier l'assemblage du cadre renfermant le milieu filtrant dans l'enveloppe extérieure.

La figure 4 représente, en vue de face et en coupe longitudinale partielle, l'unité de filtration de la figure 3 sur laquelle apparaissent les joncs d'écartement.

Une unité de filtration 1 destinée à éliminer une substance virucide présente dans un fluide biologique comprend typiquement une enveloppe extérieure 2 munie d'au moins un orifice d'entrée 3 et d'au moins un orifice de sortie 4, l'enveloppe renfermant un milieu filtrant 5 qui délimite deux compartiments respectivement d'entrée 6 et de sortie 7 de l'unité de filtration 1.

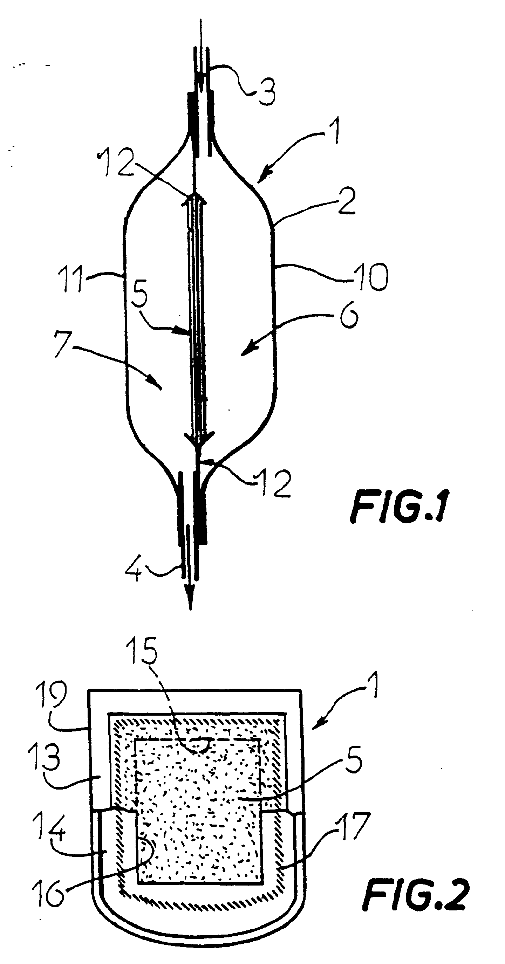

Dans la description, les termes « entrée » et « sortie » sont définis par rapport au sens de circulation du fluide biologique dans l'unité de filtration 1 (voir les flèches représentées sur la figure 1).

Selon une réalisation particulière, le fluide biologique est du sang, un composé du sang, notamment du plasma sanguin et la substance virucide est le bleu de méthylène ou l'un de ses dérivés.

Préalablement à son passage dans l'unité de filtration 1, le fluide biologique a subi un traitement d'inactivation virale au moyen de la substance virucide qui a été additionnée au fluide biologique.

Ce traitement, généralement mis en oeuvre au centre de transfusion sanguine, ne sera pas décrit plus avant ici.

L'unité de filtration 1 est destinée à être intégrée, notamment par l'intermédiaire de tubulures respectivement d'entrée 8 et de sortie 9, à un système comprenant par exemple des poches à usage médical, des tubulures, des clamps ou d'autres filtres (par exemple à déleucocyter le fluide biologique).

Dans de tel système, l'unité de filtration 1 est disposée sur le chemin d'écoulement du fluide biologique de sorte que le fluide biologique additionné de la substance virucide entre dans l'unité de filtration 1 par l'orifice d'entrée 3 et que le fluide biologique exempt de la substance virucide soit délivré par l'intermédiaire de l'orifice de sortie 4.

Un exemple particulier d'un tel système est une ligne de transfusion d'une poche contenant un fluide biologique à transfuser à un patient. Dans une telle ligne, l'unité de filtration 1 est connectée par son entrée 3 à la poche contenant le fluide biologique additionné de la substance virucide et par sa sortie 4 à des moyens de transfusion du fluide biologique exempt de substance virucide.

Ces différents systèmes ne sont pas décrits plus avant, dans la mesure où ils comprennent l'unité de filtration 1 selon la structure décrite ici.

On décrit maintenant un premier mode de réalisation de l'unité de filtration 1 comprenant une enveloppe extérieure 2 souple formée par l'assemblage de deux feuilles de matière plastique souple 10, 11 assemblées mutuellement, par exemple par soudage, sur leur périphérie (figure 1).

Cette enveloppe extérieure renferme un milieu filtrant désigné de façon générale par la référence 5, dont la structure sera décrite plus en détail dans ce qui suit.

Le milieu filtrant 5 est maintenu dans un cadre support 12 souple et étanche et délimite deux compartiments, respectivement d'entrée 6 et de sortie 7 de l'unité de filtration 1.

Le compartiment d'entrée 6 communique avec l'extérieur de l'unité de filtration 1 par l'intermédiaire d'une tubulure d'entrée 8 qui sert à son remplissage avec le fluide biologique additionné de la substance virucide.

Le compartiment de sortie 7 communique avec l'extérieur de l'unité de filtration 1 par l'intermédiaire d'une tubulure de sortie 9 qui assure la délivrance du fluide biologique exempt de substance virucide.

La structure de l'unité de filtration 1 permet ainsi au fluide biologique additionné de la substance virucide d'être reçu dans le compartiment d'entrée 6 via l'orifice d'entrée 3, de traverser le milieu filtrant 5 de sorte que la substance virucide soit absorbée et/ou adsorbée par celui-ci, puis le fluide biologique exempt de substance virucide est reçu dans le compartiment de sortie 7 pour être délivré via l'orifice de sortie 4.

Selon une forme de réalisation, les tubulures d'entrée 8 et/ou de sortie 9 sont souples, sécables et soudables.

Dans le cas où une poche de recueil est associée à la tubulure de sortie 9, cette forme de réalisation permet, après dissociation de l'unité de filtration 1 par coupure et soudage de la tubulure de sortie 9, d'obtenir une poche remplie de fluide biologique exempt de substance virucide. Une telle poche peut alors être utilisée de façon classique, par exemple pour être transfusée à un patient.

Un premier niveau d'étanchéité de l'unité de filtration 1 est assuré entre le milieu filtrant 5 et le cadre souple 12 où il n'y a aucun passage de tubulure.

Un second niveau d'étanchéité est assuré à la périphérie de l'unité de filtration 1 où les deux feuilles extérieures 10, 11, la périphérie du cadre souple 12 et le passage des tubulures d'entrée 8 et de sortie 9 sont jointes.

Ce second niveau d'étanchéité peut être assuré par les techniques connues de liaison de matériaux plastiques, par exemple par soudage à haute fréquence.

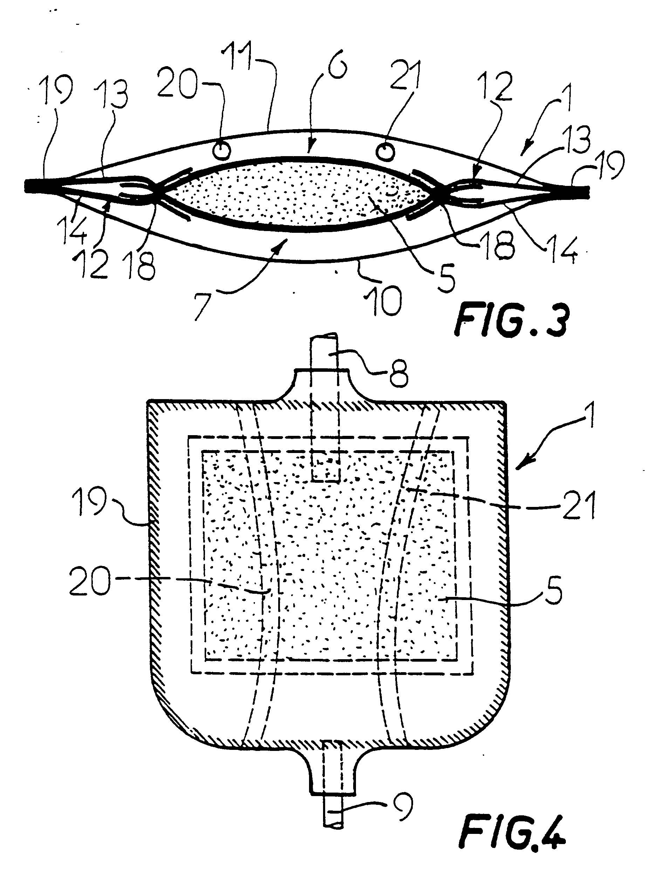

On décrit maintenant en référence aux figures 2 à 4, la réalisation de l'assemblage de l'unité de filtration 1.

Le cadre souple 12 est formé par un assemblage de deux feuilles 13, 14, par exemple plastifiées, entre lesquelles est placé le milieu filtrant 5.

Ces deux feuilles 13, 14 sont ajourées dans leur partie centrale et comportent chacune au moins une ouverture 15, 16 permettant le passage du fluide biologique à filtrer.

Les deux feuilles 13, 14 sont fixées entre elles de préférence au niveau de la périphérie du milieu filtrant 5, par exemple par un cordon de soudure 17, réalisé à travers le milieu filtrant 5, assurant à la fois la fixation du milieu filtrant 5 mais également l'étanchéité de l'unité 1.

La soudure des feuilles 13, 14 à travers le milieu filtrant 5 provoque une compression 18, formant un cordon étanche autour du milieu filtrant 5.

La périphérie 19 du cadre souple 12 est soudée également avec les feuilles extérieures 10, 11 formant l'enveloppe extérieure 2 de l'unité de filtration 1, mutuellement sur tout leur pourtour et au niveau de leur périphérie, assurant ainsi l'étanchéité de l'unité 1.

Pour éviter que le milieu filtrant 5 ne se colle contre l'enveloppe extérieure 2, et gène ainsi l'écoulement du fluide biologique dans le compartiment de sortie 7, deux joncs d'écartement 20, 21 sont placés à l'intérieur du compartiment de sortie 7, entre le milieu filtrant 5 et l'enveloppe extérieure 2.

Ces deux joncs 20, 21 dégagent le compartiment de sortie 7 du milieu filtrant 5 et évitent ainsi que le milieu filtrant 5 ne se plaque contre la paroi intérieure de la feuille extérieure 2.

Les joncs 20, 21 peuvent être réalisés à partir de tubulures souples soudées par exemple au niveau de la paroi intérieure de la feuille de l'enveloppe extérieure 2, par exemple au niveau de la soudure périphérique 19 de l'unité de filtration 1.

Il va de soi que le nombre de joncs d'écartement peut varier, en fonction par exemple des dimensions de l'unité de filtration 1.

Par exemple, il est envisageable de prévoir un jonc d'écartement unique replié de façon à former une boucle à l'intérieur du compartiment de sortie 7.

De préférence, des joncs souples sont utilisés, pour ne pas gêner les possibilités de pliage de l'unité de filtration 1.

Dans un deuxième mode de réalisation (non représenté), l'unité de filtration 1 comprend une enveloppe extérieure 2 rigide, par exemple réalisée en matériau plastique rigide tel que le polycarbonate.

On décrit maintenant plus en détail la structure et la réalisation du milieu filtrant 5 apte à éliminer sensiblement toute la substance virucide en laissant sensiblement inchangée la composition du fluide biologique lors de la filtration.

Selon l'invention, le milieu filtrant 5 est réalisé à partir d'au moins un matériau hydrophile sous la forme d'un non tissé poreux.

Additionellement, le milieu filtrant 5 est réalisé à partir d'au moins un matériau hydrophile sous la forme d'une membrane poreuse, ou

préférablement, le milieu filtrant 5 est réalisé à partir d'un matériau hydrophile sous la forme d'au moins une membrane poreuse qui est insérée entre plusieurs couches de matériau hydrophile sous la forme d'un non tissé

Dans ces trois modes de réalisation, le matériau hydrophile est apte à absorber et/ou adsorber la substance virucide, notamment par affinité entre la substance virucide et le matériau hydrophile.

Différents matériaux peuvent être utilisés pour la réalisation du milieu filtrant en fonction de la nature du fluide à filtrer et de celle du fluide biologique.

Le choix des matériaux utilisables dans l'unité de filtration selon l'invention est cependant limité par le fait qu'ils ne doivent pas empêcher, notamment par affinité, le passage des constituants cellulaires ou non cellulaires du fluide biologique.

En d'autres termes, le matériau formant le milieu filtrant doit être apte à absorber et/ou adsorber la substance virucide mais pas les constituants du fluide biologique.

Dans le cas du traitement d'un plasma sanguin contenant du bleu de méthylène, citons parmi les matériaux possibles, les polymères et/ou les copolymères à base de polyester, d'acrylonitrile, de fluorure de polyvinylidène.

Ces produits polymériques ne sont généralement pas hydrophiles naturellement et doivent être traités par des méthodes physiques et/ou chimiques, pour leur conférer lesdites propriétés hydrophiles.

Ces traitements consistent par exemple dans le greffage de substituants hydrophiles, par exemple des groupements de type hydroxyle ou carboxylique, sur le polymère, selon des méthodes connues.

De tels polymères rendus hydrophiles par traitement physique et/ou chimique sont disponibles sur le marché.

Le caractère hydrophile du matériau formant le milieu filtrant 5 permet une bonne mouillabilité du milieu filtrant lors du passage du fluide biologique, ce qui permet notamment un meilleur écoulement du fluide biologique à travers l'unité de filtration 1 mais également d'améliorer l'efficacité de la filtration.

Les caractéristiques de porosité du milieu filtrant permettent le passage du fluide biologique à travers l'unité de filtration en laissant sensiblement inchangée la composition du fluide biologique.

A cet effet, la taille moyenne des pores du milieu filtrant est choisie en fonction du fluide biologique à traiter. Par exemple pour que l'unité de filtration 1 laisse passer les constituants du sang entier, la taille moyenne des pores peut être de l'ordre ou supérieure à 7 µm. Dans le cas d'un plasma sanguin, la taille moyenne des pores peut être plus faible, par exemple de l'ordre de 4 µm, du fait de l'absence de constituants cellulaires dans le plasma.

Lors du passage du fluide biologique additionné de la substance virucide au travers du milieu filtrant 5, la surface de contact entre le fluide biologique et le milieu filtrant doit être suffisante pour éliminer sensiblement toute la substance virucide en laissant sensiblement inchangée la composition du fluide biologique.

Dans le premier mode de réalisation, cette caractéristique est avantageusement obtenue grâce à l'utilisation d'un matériau non tissé qui présente, de par sa structure, une grande surface de contact pour un faible volume.

Par surface de contact entre le fluide biologique et le milieu filtrant, on entend la surface sur laquelle l'absorption et/ou l'adsorption de la substance virucide par le matériau poreux peut avoir lieu. Il va de soit que cette surface est fonction notamment de la surface du milieu filtrant, de sa porosité, de son épaisseur, du diamètre des fibres du non tissé.

Ainsi, en modifiant le diamètre des fibres, la porosité du matériau non tissé et l'épaisseur du milieu filtrant 5 qu'il compose, on peut avoir accès à une large gamme de surface de contact qui permet d'éliminer sensiblement toute la substance virucide en laissant sensiblement inchangée la composition du fluide biologique.

A titre d'exemple, on peut citer un milieu filtrant 5 formé d'un non tissé en polyester ayant une épaisseur de l'ordre de 5 mm, une porosité moyenne de l'ordre de 8 µm et un diamètre moyen des fibres de l'ordre de 2 µm, permettant l'élimination d'une concentration de 1 µM de bleu de méthylène dans 250 ml de plasma sanguin.

Notons toutefois que ces valeurs peuvent varier dans une large mesure, notamment en fonction du temps de contact entre le milieu filtrant et le fluide biologique, c'est à dire de la vitesse de filtration.

Additionnellement une membrane poreuse est utilisée comme milieu filtrant 5 pour absorber et/ou adsorber la substance virucide présente dans le fluide biologique.

Dans un exemple particulier, une telle membrane est réalisée en fluorure de polyvinylidène et à une taille de pores calibrée à une valeur comprise entre 1 et 15 µm.

Dans le troisième mode de réalisation, le milieu filtrant 5 peut combiner les deux matériaux utilisés dans les modes de réalisation précédents, à savoir comprendre plusieurs couches de matériau hydrophile sous la forme d'un non tissé poreux et d'une ou plusieurs membranes poreuses. Le matériau et/ou la structure du matériau formant ces couches peut alors être identique ou différent entre eux.

Les couches sont alors disposées, par exemple de façon jointive, les unes à côté des autres dans l'unité de filtration de sorte que le fluide biologique les traversent successivement lors de la filtration.

Dans un exemple particulier, on peut citer un milieu filtrant 5 formé d'une superposition de couches formées respectivement : d'un non tissé polyester de type « spunbond », d'un non tissé polyester de type « meltblown », d'une ou plusieurs membranes en fluorure de polyvinylidène, d'un non tissé polyester de type « meltblown » et d'un non tissé polyester de type « spunbond ».

Par les termes « spunbond » et « meltblown », on entend deux des méthodes classiques de formation d'une couche de non tissé directement à partir du polymère, à savoir respectivement soit en formant des monofilaments continus soit en soufflant le polymère à l'état fondu en filaments irréguliers.

Ces techniques étant classiques, nous ne les détaillerons pas plus ici.

Dans cette réalisation, les deux couches extérieures de non tissé « spunbond » sont identiques et servent respectivement de pré et de post filtre. De plus, elles ont pour fonction d'améliorer la soudabilité du milieu filtrant 5 sur l'enveloppe 2 de l'unité de filtration 1.

Les deux couches de non tissé « meltblown » ainsi que la ou les membranes placées entre elles forment plus particulièrement le milieu filtrant 5 apte à absorber et/ou adsorber la substance virucide..

En outre, les deux couches de non tissé « meltblown » sont identiques et ont pour fonction de protéger la ou les membranes.

## Revendications

1. Unité de filtration (1) destinée à éliminer une substance virucide présente dans un fluide biologique, comprenant une enveloppe extérieure (2) munie d'au moins un orifice d'entrée (3) et d'au moins un orifice de sortie (4), l'enveloppe (2) renfermant un milieu filtrant (5) qui délimite deux compartiments respectivement d'entrée (6) et de sortie (7) de l'unité de filtration (1), **caractérisée en ce que** le milieu filtrant est réalisé à partir d'au moins un matériau hydrophile sous la forme d'un non tissé poreux et/ou d'une membrane poreuse apte à absorber et/ou adsorber la substance virucide.

2. Unité de filtration selon la revendication 1, **caractérisée en ce que** le milieu filtrant comprend en outre au moins un matériau hydrophile sous la forme d'une membrane poreuse apte à absorber et/ou adsorber la substance virucide.

3. Unité de filtration (1) selon la revendication 1 ou 2, **caractérisée en ce que** la porosité moyenne du milieu filtrant (5) est définie de sorte que la surface de contact entre le fluide biologique et le milieu filtrant soit suffisante pour éliminer sensiblement toute la substance virucide en laissant sensiblement inchangée la composition du fluide biologique lors de son passage à travers le milieu filtrant (5), à savoir étant comprise entre 1 µm et 15 µm.

4. Unité de filtration (1) selon l'une quelconque des revendications 1 à 3, **caractérisé en ce que** le diamètre moyen des fibres du non tissé poreux est compris entre 0,5 µm et 5 µm.

5. Unité de filtration (1) selon l'une quelconque des revendications 1 à 4, **caractérisée en ce que** le compartiment d'entrée (6) communique avec l'extérieur de l'unité de filtration (1) par l'intermédiaire d'une tubulure d'entrée (8).

6. Unité de filtration (1) selon l'une quelconque des revendications 1 à 5, **caractérisée en ce que** le compartiment de sortie (7) communique avec l'extérieur de l'unité de filtration (1) par l'intermédiaire d'une tubulure de sortie (9).

7. Unité de filtration (1) selon la revendication 5 ou 6, **caractérisée en ce que** les tubulures d'entrée (8) et de sortie (9) sont souples, sécables et soudables.

8. Unité de filtration (1) selon l'une quelconque des revendications 1 à 7, **caractérisée en ce que** le matériau hydrophile du milieu filtrant est choisi notamment parmi les matériaux hydrophiles naturellement ou les matériaux, notamment à base de matière plastique, rendu hydrophiles.

9. Unité de filtration (1) selon la revendication 8, **caractérisée en ce que** le matériau hydrophile est choisi notamment parmi les polymères et/ou les copolymères à base de polyester, d'acrylonitrile, de fluorure de polyvinylidène.

10. Unité de filtration (1) selon l'une quelconque des revendications 1 à 9, **caractérisée en ce que** le milieu filtrant (5) comprend plusieurs couches de matériau hydrophile, de nature identique ou différente entre eux, de surface de contact identique ou différente entre eux.

11. Unité de filtration (1) selon l'une quelconque des revendications 1 à 10, **caractérisée en ce que** le milieu filtrant (5) possède une épaisseur comprise entre 1 et 10 millimètres.

12. Unité de filtration (1) selon l'une quelconque des revendications 1 à 11, **caractérisée en ce que** l'enveloppe extérieure (2) est rigide.

13. Unité de filtration (1) selon l'une quelconque des revendications 1 à 11, **caractérisée en ce que** l'enveloppe extérieure (2) est souple.

14. Unité de filtration (1) selon la revendication 13, **caractérisée en ce que** l'enveloppe souple (2) est formée de deux feuilles de matière plastique (10, 11) souple assemblée sur leur périphérie, le milieu filtrant (5) étant maintenu dans un cadre (12) souple et étanche délimitant avec le milieu filtrant (5) les compartiments d'entrée (6) et de sortie (7) de l'unité de filtration (1).

15. Unité de filtration (1) selon la revendication 14, **caractérisée en ce que** le cadre souple (12) est formé de deux feuilles (13, 14) souples ajourées entre elles entre lesquelles le milieu filtrant (5) est placé.

16. Unité de filtration (1) selon la revendication 15, **caractérisée en ce que** les feuilles (13, 14) formant le cadre souple (12) sont fixées entre elles au niveau de la périphérie du milieu filtrant (5) et également avec les feuilles (10, 11) formant l'enveloppe extérieure (2), au niveau de la périphérie de l'enveloppe extérieure (2) de l'unité de filtration (1).

17. Unité de filtration (1) selon la revendication 15 ou 16, **caractérisée en ce que** la fixation des feuilles (13, 14) formant le cadre souple (12) est un cordon de soudure (17) réalisé à travers le milieu filtrant (5).

18. Unité de filtration (1) selon l'une quelconque des revendications 13 à 17, **caractérisée en ce que** le compartiment de sortie (7) est dégagé du milieu filtrant (5) par la présence d'un ou plusieurs joncs d'écartement (20, 21) disposés entre le milieu filtrant (5) et l'enveloppe extérieure (2) souple, à l'intérieur du compartiment de sortie (7).

19. Unité de filtration (1) selon la revendication 18, **caractérisée en ce que** le ou les joncs d'écartement (20, 21) sont réalisés à partir de tubulures souples soudées par exemple au niveau de la paroi interne de la feuille de l'enveloppe extérieure (2).

20. Unité de filtration (1) selon l'une quelconque des revendications 1 à 19, **caractérisée en ce que** la substance virilucide est le bleu de méthylène ou l'un de ses dérivés.

21. Unité de filtration (1) selon l'une quelconque des revendications 1 à 20, **caractérisée en ce que** le fluide biologique est du sang, un composé du sang, notamment du plasma sanguin.

## Claims

1. A filtration unit (1) designed to remove a virucidal substance that is present in a biological fluid, said filtration unit comprising an outer casing (2) provided with at least one inlet orifice (3) and with at least one outlet orifice (4), the casing (2) enclosing a filtering medium (5) which defines two compartments, respectively an inlet compartment (6) and an outlet compartment (7), of the filtration unit (1), said filtration unit being **characterized in that** the filtering medium is formed from at least one hydrophilic material in the form of a porous non-woven fabric suitable for absorbing and/or adsorbing the virucidal substance.

2. A filtration unit according to claim 1, **characterized in that** the filtering medium further comprises at least one hydrophilic material in the form of a porous membrane suitable for absorbing and/or adsorbing the virucidal substance.

3. A filtration unit (1) according to claim 1 or claim 2, **characterized in that** the mean porosity of the filtering medium (5) is defined so that the surface area of contact between the biological fluid and the filtering medium is sufficient to remove substantially all of the virucidal substance while leaving the composition of the biological fluid substantially unchanged on passing through the filtering medium (5) namely said mean porosity lies in range 1 µm to 15 µm.

4. A filtration unit (1) according to any one of claims 1 to 3, **characterized in that** the mean diameter of the fibers of the porous non-woven fabric lies in the range 0.5 µm to 5 µm.

5. A filtration unit (1) according to any one of claims 1 to 4, **characterized in that** the inlet compartment (6) communicates with the outside of the filtration unit (1) via an inlet connection pipe (8).

6. A filtration unit (1) according to any one of claims 1 to 5, **characterized in that** the outlet compartment (7) communicates with the outside of the filtration unit (1) via an outlet connection pipe (9).

7. A filtration unit (1) according to claim 5 or claim 6, **characterized in that** the inlet connection pipe (8) and the outlet connection pipe (9) are flexible, breakable, and sealable or otherwise bondable.

8. A filtration unit (1) according to any one of claims 1 to 7, **characterized in that** the hydrophilic material of the filtering medium is chosen, in particular, from materials that are naturally hydrophilic, or materials, in particular based on plastics, that are made hydrophilic.

9. A filtration unit (1) according to claim 8, **characterized in that** the hydrophilic material is chosen in particular from polymers and/or copolymers based on polyester, on acrylonitrile, and on polyvinylidene fluoride.

10. A filtration unit (1) according to any one of claims 1 to 9, **characterized in that** the filtering medium (5) comprises a plurality of layers of hydrophilic material that are of mutually identical type or of mutually different types, and that are of mutually identical contact surface area or of mutually different contact surface areas.

11. A filtration unit (1) according to any one of claims 1 to 10, **characterized in that** the filtration medium (5) has a thickness lying in the range 1 millimeter to 10 millimeters.

12. A filtration unit (1) according to any one of claims 1 to 11, **characterized in that** the outer casing (2) is rigid.

13. A filtration unit (1) according to any one of claims 1 to 11, **characterized in that** the outer casing (2) is flexible.

14. A filtration unit (1) according to claim 13, **characterized in that** the flexible casing (2) is made up of two sheets of flexible plastics material (10, 11) assembled together around their peripheries, the filtering medium (5) being held in a flexible and leaktight frame (12) cooperating with the filtering medium (5) to define the inlet compartment (6) and the outlet compartment (7) of the filtration unit (1).

15. A filtration unit (1) according to claim 14, **characterized in that** the flexible frame (12) is made up of two non-touching flexible sheets (13, 14) between which the filtering medium (5) is placed.

16. A filtration unit (1) according to claim 15, **characterized in that** the sheets (13, 14) forming the flexible frame (12) are fastened to each other at the periphery of the filtering medium (5) and also to the sheets (10, 11) forming the outer casing (2), at the periphery of the outer casing (2) of the filtration unit (1).

17. A filtration unit (1) according to claim 15 or claim 16, **characterized in that** the sheets (13, 14) forming the flexible frame (12) are fastened by means of a bead of sealing or of other bonding (17) formed through the filtering medium (5).

18. A filtration unit (1) according to any one of claims 13 to 17, **characterized in that** the outlet compartment (7) is spaced apart from the filtering medium (5) being the presence of one or more spacer rods (20, 21) disposed between the filtering medium (5) and the flexible outer casing (2), inside the outlet compartment (7).

19. A filtration unit (1) according to claim 18, **characterized in that** the spacer rods (20, 21) are formed from flexible connection pipes sealed or otherwise bonded, for example, to the inside wall of the sheet of the outer casing. (2).

20. A filtration unit (1) according to any one of claims 1 to 19, **characterized in that** the virucidal substance is methylene blue or one of the derivatives thereof.

21. A filtration unit (1) according to any one of claims 1 to 20, **characterized in that** the biological fluid is blood, or a component of blood, in particular blood plasma.

## Patentansprüche

1. Filtereinheit (1), die dazu bestimmt ist, eine in einem biologischen Fluid vorhandene virustötende Substanz auszufiltern, die einen äußeren Mantel (2) umfaßt, der mit mindestens einer Eintrittsöffnung (3) und mindestens einer Auslaßöffnung (4) versehen ist, wobei der Mantel (2) ein Filtermedium (5) umfaßt, der zwei Abteile für den Eintritt (6) beziehungsweise für den Auslaß (7) der Filtereinheit (1) abgrenzt, **dadurch gekennzeichnet, daß** das Filtermedium ausgehend von mindestens einem hydrophilen Material in Form eines Porenvlieses ausgeführt ist, das fähig ist, die virustötende Substanz zu absorbieren und/oder zu adsorbieren.

2. Filtereinheit (1) nach Anspruch 1, **dadurch gekennzeichnet, daß** das Filtermedium außerdem mindestens ein hydrophilen Material in Form von einer porösen Membran umfaßt, die fähig ist, die virustötende Substanz zu absorbieren und/oder zu adsorbieren.

3. Filtereinheit (1) nach Anspruch 1 oder 2, **dadurch gekennzeichnet, daß** die durchschnittliche Porosität des Filtermediums (5) so definiert ist, daß die Kontaktfläche zwischen dem biologischen Fluid und dem Filtermedium ausreicht, um deutlich die gesamte virustötende Substanz auszufiltern und dabei die Zusammensetzung des biologischen Fluids bei seinem Durchlauf durch das Filtermedium (5) deutlich unverändert zu lassen, nämlich zwischen 1 µm und 15 µm.

4. Filtereinheit (1) nach einem beliebigen der vorstehenden Ansprüche 1 bis 3, **dadurch gekennzeichnet, daß** der durchschnittliche Durchmesser der Fasern des porösen Vliesmaterials zwischen 0,5 µm und 5 µm liegt.

5. Filtereinheit (1) nach einem beliebigen der vorstehenden Ansprüche 1 bis 4, **dadurch gekennzeichnet, daß** das Eintrittsabteil (6) über einen Eintrittsstutzen (8) mit dem Äußeren der Filtereinheit (1) im Verbindung steht.

6. Filtereinheit (1) nach einem beliebigen der vorstehenden Ansprüche 1 bis 5, **dadurch gekennzeichnet, daß** das Auslaßabteil (7) über einen Auslaßstutzen (9) mit dem Äußeren der Filtereinheit (1) im Verbindung steht.

7. Filtereinheit (1) nach Anspruch 5 oder 6, **dadurch gekennzeichnet, daß** die Eintritts- (8) und Auslaßstutzen (9) weich, trennbar und schweißbar sind.

8. Filtereinheit (1) nach einem beliebigen der vorstehenden Ansprüche 1 bis 7, **dadurch gekennzeichnet, daß** das hydrophile Material des Filtermediums insbesondere unter den naturbedingt hydrophilen Stoffen oder hydrophil gemachten Stoffen insbesondere auf Kunststoffbasis bestehen.

9. Filtereinheit (1) nach Anspruch 8, **dadurch gekennzeichnet, daß** das hydrophile Material insbesondere unter den Polymeren und/oder den Copolymeren auf Grundlage von Polyester, Acrylonitril, Polyvinylidenfluorid gewählt wird.

10. Filtereinheit (1) nach einem beliebigen der vorstehenden Ansprüche 1 bis 9, **dadurch gekennzeichnet, daß** das Filtermedium (5) mehrere Schichten identischen oder verschiedenen hydrophilen Materials mit identischer oder untereinander verschiedener Kontaktfläche umfaßt.

11. Filtereinheit (1) nach einem beliebigen der vorstehenden Ansprüche 1 bis 10, **dadurch gekennzeichnet, daß** das Filtermedium (5) eine Dicke von 1 bis 10 Millimeter umfaßt.

12. Filtereinheit (1) nach einem beliebigen der vorstehenden Ansprüche 1 bis 11, **dadurch gekennzeichnet, daß** der äußere Mantel (2) starr ist.

13. Filtereinheit (1) nach einem beliebigen der vorstehenden Ansprüche 1 bis 11, **dadurch gekennzeichnet, daß** der äußere Mantel (2) weich ist.

14. Filtereinheit (1) nach Anspruch 13, **dadurch gekennzeichnet, daß** der weiche Mantel (2) aus zwei weichen an ihrer Peripherie verbundenen Kunststoffolien (10, 11) gebildet wird, wobei das Filtermedium (5) in einem weichen und dichten Rahmen (12) gehalten wird, der mit dem Filtermedium (5) das Eintritts- (6) und das Auslaßabteil (7) der Filtereinheit abgrenzt.

15. Filtereinheit (1) nach Anspruch 14, **dadurch gekennzeichnet, daß** der weiche Rahmen (12) aus zwei weichen durchbrochenen Folien (13, 14) gebildet wird, zwischen denen das Filtermedium (5) eingeführt wird.

16. Filtereinheit (1) nach Anspruch 15, **dadurch gekennzeichnet, daß** die Folien (13, 14), die den weichen Rahmen (12) bilden, an der Peripherie des Filtermediums (5) miteinander befestigt sind und auch mit den Folien (10, 11), die den äußeren Mantel (2) bilden, und zwar an der Peripherie des äußeren Mantels (2) der Filtereinheit (1).

17. Filtereinheit (1) nach Anspruch 15 oder 16, **dadurch gekennzeichnet, daß** die Befestigung der Folien (13, 14), die den weichen Rahmen (12) bilden, ein Schweißwulst (17) ist, der durch das Filtermedium (5) hindurch gebildet wird.

18. Filtereinheit (1) nach einem beliebigen der vorstehenden Ansprüche 13 bis 17, **dadurch gekennzeichnet, daß** das Auslaßabteil (7) durch einen oder mehrere vorhandene Abstandsringe (20, 21) vom Filtermedium (5) gelöst ist, die zwischen dem Filtermedium (5) und dem äußeren weichen Mantel (2) innen im Auslaßabteil (7) angeordnet sind.

19. Filtereinheit (1) nach Anspruch 18, **dadurch gekennzeichnet, daß** der oder die Abstandsring/e (20, 21) ausgehend von weichen Stutzen ausgeführt sind, die zum Beispiel an die innere Wand der Folie des äußeren Mantels (2) geschweißt sind.

20. Filtereinheit (1) nach einem beliebigen der vorstehenden Ansprüche 1 bis 19, **dadurch gekennzeichnet, daß** die virustötende Substanz das Methylenblau oder eines seiner Derivate ist.

21. Filtereinheit (1) nach einem beliebigen der vorstehenden Ansprüche 1 bis 20, **dadurch gekennzeichnet, daß** das biologische Fluid Blut ist, eine Komponente von Blut und insbesondere Blutplasma ist.
